# EUROPÄISCHE PATENTSCHRIFT

(11) **EP 2 918 177 B1**
(45) Veröffentlichungstag und Bekanntmachung des Hinweises auf die Patenterteilung: **27.02.2019**
(21) Anmeldenummer: 14000865.7
(22) Anmeldetag: 11.03.2014
(51) Int. Cl.: A23L 33/15, A23L 33/16, A23L 33/175, A61K 31/198, A61P 25/20, A61P 25/28

(54) **Nahrungsergänzungsmittel zur Schlafoptimierung**
Food supplement for sleep optimisation
Complément alimentaire pour l'optimisation du sommeil

(43) Veröffentlichungstag der Anmeldung: 16.09.2015
(73) Patentinhaber: Third of Life GmbH, 81929 München (DE)
(72) Erfinder: DEYLE, Hanno-Götz, Dr., 80636 München (DE); Kuhn, Frieder, 81929 München (DE); DWORAK, Markus, Dr., 90419 Nürnberg (DE)
(74) Vertreter: Weigel, Matthias

(56) Entgegenhaltungen:
- EP-A1- 1 190 628
- EP-A1- 2 377 578
- WO-A1-03/047367
- WO-A2-03/101402
- DE-A1- 19 929 995
- DE-A1-102007 030 495
- DE-A1-102007 062 288
- US-A1- 2012 178 799
- DATABASE WPI Week 201414 Thomson Scientific, London, GB; AN 2014-A64519 XP002726178, & CN 103 371 317 A (SHANDONG BANGAO VENTURE BIO-TECHNOLOGY) 30. Oktober 2013 (2013-10-30) & CN 103 371 317 A (SHANDONG BANGAO ENTREPRENEURIAL BIOTECHNOLOGY CO LTD) 30. Oktober 2013 (2013-10-30)
- DATABASE WPI Week 200532 Thomson Scientific, London, GB; AN 2005-310239 XP002726179, & JP 2005 097161 A (NISSHIN PHARMA KK) 14. April 2005 (2005-04-14) & JP 2005 097161 A (NISSHIN PHARMA INC) 14. April 2005 (2005-04-14)
- Ingraham, Paul: "Save yourself from Insomnia!", anonymous , 11. Dezember 2013 (2013-12-11), Seiten 1-15, XP002726180, Gefunden im Internet: URL:http://saveyourself.ca/articles/insomn ia.php [gefunden am 2014-06-24]

## Beschreibung

Schlaf ist für die Funktionsfähigkeit des Gehirns und das Überleben des gesamten Organismus notwendig und demnach für das menschliche Leben von vitaler Bedeutung. Jeder zweite Deutsche klagt mittlerweile über mangelnde Erholung im Schlaf, obwohl nur etwa eine Million Deutsche ernsthafte Schlafstörungen aufweisen. Schlafprobleme führen zu Tagesmüdigkeit, schlechter Konzentration und fördern langfristig die Entstehung von Übergewicht, Diabetes und Herz-Kreislauf-Erkrankungen.

Die Ursachen von Schlafstörungen können vielseitig und komplex sein. Einschlaf und Durchschlafstörungen können in diesem Zusammenhang genannt werden. Auch eine geringe Schlafqualität kann zur Tagesmüdigkeit führen, selbst wenn keine klinisch relevanten Einschlaf- oder Durchschlafstörungen vorliegen.

Schlaf ist ein lebensnotwendiger, aktiver Prozess, bei dem in den Organen Regenerations- und Aufbauprozesse ablaufen. Der Schlaf dient unter anderem der Wiederherstellung von Energiereserven (beispielsweise im Gehirn) und der Zellerneuerung. Hinsichtlich der möglichen Funktionen des Schlafs wird daher in der regenerativen Hypothese davon ausgegangen, dass Schlaf der Erholung der Organe dient. Die verbesserte Effizienz dieser Regenerationsprozesse kann wiederum die Schlafqualität steigern, so dass der Schlaf als deutlich erholsamer empfunden wird.

Das durchschnittliche Schlafbedürfnis erwachsener Personen beträgt etwa 6-9 Stunden. Geringere Schlafzeiten bzw. Schlafmangel, beispielsweise bedingt durch eine berufliche Belastung oder eine Reisebelastung (z.B. Jetlag), können auch bei schlafgesunden Personen die Leistungsfähigkeit am nächsten Tag nachteilig beeinflussen und zu einer Tagesmüdigkeit führen. Dabei gilt, dass die aus dem Schlafinangel resultierende Abnahme der Leistungsfähigkeit oder Wachheit (Vigilanz) bzw. Zunahme der Tagesschläfrigkeit umso ausgeprägter ist, je geringer die Schlafqualität und der aus dem Schlaf resultierende Erholungseffekt ist. Der Begriff "Schlafqualität" ist ein in der Schlafmedizin etablierter Begriff. Für die Bewertung der Schlafqualität existieren etablierte Methoden, wie z.B. der Pittsburgh Schlafqualitätsindex PSQI ("Pittsburgh Sleep Quality Index"). Auch die Tagesschläfrigkeit bzw. Tagesmüdigkeit ist ein anerkannter Begriff in der Schlafmedizin, für die etablierte Bewertungsmethoden wie z.B. die Pupillographie existieren. Vigilanz ist der Fachausdruck für die Wachheit einer Person. Diese kann anhand etablierter Vigilanztests bestimmt werden. Solche Vigilanztests werden beispielsweise am Computer ausgeführt, wobei die Fähigkeit der Testperson, auch in monotonen und lange andauernden Situationen auf seltene Reize angemessen zu reagieren, untersucht wird.

Es existieren pharmakologisch wirkende Schlafmittel, die die Entspannung fördern oder den Schlaf künstlich herbeiführen. Diese Schlafmittel beeinflussen den Schlaf auf unnatürliche Weise, sind z.T. mit starken Nebenwirkungen und der Gefahr der Abhängigkeit verbunden und zudem nur für eine limitierte Personengruppe zu empfehlen. Bisher gibt es keine Nährstoffkombination, die auf natürliche Weise den Schlaf optimiert, die nächtliche Regeneration fördert und die Leistungsfähigkeit am nächsten Tag verbessert.

DE 10 2007 030495 A1 beschreibt die Verwendung einer eine Kreatin-Komponente enthaltenden Zubereitung zur Verbesserung der Gedächtnisleistung, der Merkfähigkeit, des Langzeitgedächtnisses und zur Vorbeugung geistiger Ermüdungszustände.

EP 1 190 628 A1 beschreibt ein Nahrungsergänzungsmittel, das Kreatin, Ginseng und Astragalus sowie optional Glutamin enthält.

CN 103 371 317 A beschreibt ein Nahrungsergänzungsmittel auf Basis von D-Ribose. Als weitere Bestandteile werden unter anderem Kreatin, Glycin und Glutamin erwähnt.

JP 2005-097161 beschreibt eine Zusammensetzung, die gegen Müdigkeit verwendet werden kann und Coenzym Q10 enthält.

DE 199 29 995 A1 beschreibt die Verwendung von Kreatin und/oder Kreatin-Derivaten zur Behandlung von Befindlichkeitsstörungen bei Frauen.

Hieraus ergibt sich der Bedarf für ein Nahrungsergänzungsmittel, welches die natürlichen Funktionen des Schlafes unterstützt, frei von Nebenwirkungen ist und für alle Personengruppen uneingeschränkt anwendbar ist. Insbesondere soll es auch die nächtliche Erholung und Schlafqualität bzw. Schlafeffizienz der normal Schlafenden (schlafgesunde Personen) optimieren.

Gelöst wird diese Aufgabe durch ein Nahrungsergänzungsmittel, das Kreatin, Glutamin und Glycin enthält, zur Verwendung als natürliches Schlafmittel.

Durch diese abgestimmte Nährstoffkombination wird die Regeneration im Schlaf gefördert, der Schlaf optimiert und die negativen Auswirkungen von mangelndem Schlaf reduziert. Die im Schlaf ablaufenden Erholungsprozesse werden beschleunigt und optimiert.

Die spezifische Kombination der ausgewählten Nährstoffe weist eine gezielte Wechselwirkung auf und verstärkt den Effekt der Einzelsubstanzen. Kreatin unterstützt die Regeneration der zellulären Energiespeicher, vor allem durch die Bildung von Adenosin-Triphosphat (ATP). Hohe ATP-Konzentrationen fördern die Proteinsynthese und die Zellregeneration, z.T. durch die Aktivierung der Adenosin-Monophosphat aktivierten Proteinkinase (AMPK) (siehe Dworak et al., J Neurosci. 2010; 30(26):9007-16). Glutamin ist in diesem Schritt für die Bildung neuer Proteine und die Zellregeneration wesentlich und dadurch ein wichtiger Bestandteil schlafspezifischer Regenerationsprozesse. Glutamin fördert die Ausschüttung des Wachstumshormons GH, welches vor allem im Schlaf freigesetzt wird und eine zentrale Bedeutung bei der Regeneration hat. Weiterhin kann im Körper Glutamin zusätzlich für die Bildung des schlaffördernden Botenstoffes GABA (Gamma-Amino-Buttersäure) herangezogen werden, wodurch die Entspannung gefördert und das Einschlafen erleichtert wird.

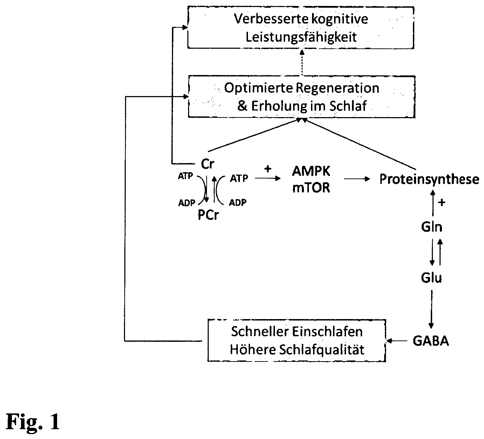

Das spezifische Zusammenspiel von Kreatin und Glutamin bei der Verstärkung bzw. Unterstützung der im Schlaf stattfindenden Regenerationsprozesse ist in Figur 1 schematisch dargestellt. Wie Figur 1 veranschaulicht, fördert und beschleunigt Kreatin (Cr) die Wiederauffüllung zellulärer Energiespeicher in Form von ATP. "PCr" steht in Figur 1 für Phospho-Kreatin. Hohe zelluläre ATP-Konzentrationen fördern die Proteinsynthese u.a. durch Adenosin-Monophosphat aktivierte Proteinkinase (AMPK) und den sogenannten "mTOR (ammalian target of rapamycin) Pathway". Glutamin (Gln) wiederum unterstützt den Zellstoffwechsel und die Proteinsynthese. Weiterhin kann, wie bereits oben erwähnt, Glutamin im Körper zusätzlich (über die Bildung von Glutamat (Glu)) für die Bildung des schlaffördernden Botenstoffes GABA herangezogen werden.

Durch diese spezifische Kombination von Kreatin und Glutamin bewirkt das erfindungsgemäße Nahrungsergänzungsmittel eine optimierte Regeneration und Erholung im Schlaf und eignet sich daher zur Verwendung bei der Schlafoptimierung (insbesondere der Verbesserung der Schlafqualität) und der Verbesserung der schlafspezifischen Regeneration.

Außerdem enthält das Nahrungsergänzungsmittel noch Glycin.

Ähnlich wie Glutamin ist auch Glycin an dem Schritt für die Bildung neuer Proteine und der Zellregeneration beteiligt und kann daher im Zusammenspiel mit Glutamin und Kreatin die schlafspezifischen Regenerationsprozesse weiter verstärken. Auch Glycin fördert die Ausschüttung des Wachstumshormons GH, welches vor allem im Schlaf freigesetzt wird und eine zentrale Bedeutung bei der Regeneration hat.

In einer bevorzugten Ausführungsform enthält das Nahrungsergänzungsmittel außerdem noch einen oder mehrere Mineralstoffe (auch als anorganische Nährstoffe bezeichnet). Als bevorzugter Mineralstoff kann insbesondere Magnesium genannt werden. Das Magnesium kann in Form üblicher Salze in dem Nahrungsergänzungsmittel vorliegen. Der Mineralstoff Magnesium ist ein zentrales Element im Energiestoffwechsel des Körpers und spielt als Coenzym im Stoffwechsel von vielen Proteinen eine wichtige Rolle. Da Magnesium die Funktion von Glutamin und Glycin im Proteinstoffwechsel unterstützt, optimiert es die Regeneration im Schlaf zusätzlich.

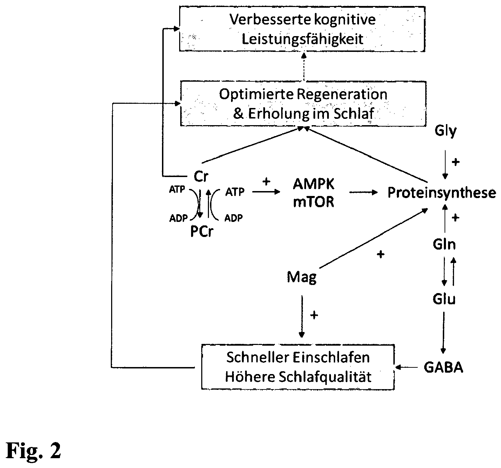

Die sich durch die zusätzliche Anwesenheit von Glycin und Magnesium ergebenden Wechselwirkungen der Komponenten des Nahrungsergänzungsmittels sind in Figur 2 schematisch dargestellt. Glycin (Gly) und Magnesium (Mag) unterstützen das Glutamin bei der Proteinsynthese. Weiterhin wirkt Magnesium (wie auch GABA, das über Glutamin gebildet werden kann) schlaffördernd.

Das Nahrungsergänzungsmittel kann außerdem ein oder mehrere Vitamine aus der Vitamin-B-Gruppe enthalten. Beispielhaft können Vitamin B1, Vitamin B2, Nicotinsäure (auch als Niacin oder Vitamin B3 bezeichnet) oder eines ihrer Salze, Pantothensäure (auch als Vitamin B5 bezeichnet) oder eines ihrer Salze, Vitamin B6, Vitamin B7 (auch als Biotin bezeichnet) oder Gemische aus mindestens zwei dieser Verbindungen genannt werden. Bevorzugt enthält das Nahrungsergänzungsmittel zumindest die folgenden Vitamine der Vitamin-B-Gruppe: Vitamin B1, Vitamin B2, Vitamin B3, Vitamin B5, Vitamin B6 und Vitamin B7.

Optional kann das Nahrungsergänzungsmittel noch weitere Vitamine wie z.B. Vitamin C enthalten.

Das Nahrungsergänzungsmittel kann außerdem noch ein oder mehrere Spurenelemente enthalten. In einer bevorzugten Ausführungsform handelt es sich bei dem Spurenelement um Zink. Das Zink kann in Form üblicher Salze in dem Nahrungsergänzungsmittel vorliegen.

Zusätzlich zu dem Glutamin und dem Glycin kann das Nahrungsergänzungsmittel noch weitere proteinogene alpha-Aminosäuren enthalten. Beispielhaft können in diesem Zusammenhang Alanin, Arginin, Asparaginsäure, Cystein, Histidin, Prolin, Serin, Threonin, Tryptophan, Tyrosin, Valin oder Gemisch aus zwei oder mehr dieser Aminosäuren genannt werden.

Das Nahrungsergänzungsmittel kann außerdem noch gamma-Aminobuttersäure (GABA) enthalten.

Weiterhin kann das Nahrungsergänzungsmittel noch ein oder mehrere Pflanzenextrakte wie z.B. ein Hopfenextrakt oder ein Extrakt der Passionsblume enthalten.

Die Zusammensetzung der vorliegenden Erfindung enthält keine synthetischen Schlafmittel.

Das Nahrungsergänzungsmittel kann in unterschiedlichen Darreichungsformen bereit gestellt werden, die dem Fachmann grundsätzlich bekannt sind. Beispielsweise kann es in Form eines Pulvers oder eines (Trink-)Granulates oder auch als flüssige Zusammensetzung (z.B. durch Auflösen des Pulvers oder Granulats in Wasser) vorliegen.

Erfindungsgemäß wird das oben beschriebene Nahrungsergänzungsmittel als natürliches Schlafmittel verwendet.

Bevorzugt erfolgt die Schlafoptimierung durch Verbesserung der Schlafqualität.

Die Verbesserung der Schlafqualität erfolgt bevorzugt durch eine Beschleunigung der im Schlaf ablaufenden Regenerationsprozesse im menschlichen Körper.

Wie bereits oben erläutert, existieren etablierte Methoden zur Bewertung der Schlafqualität (z.B. der Pittsburgh Schlafqualitätsindex PSQI ("Pittsburgh Sleep Quality Index")), der Tagesschläfrigkeit bzw. Tagesmüdigkeit (z.B. Pupillographie), der Vigilanz (z.B. Computer-basierte Aufmerksamkeitstests) und kognitiven Leistungsfähigkeit (z.B. Tests zur Bewertung der Konzentrationsfähigkeit). Die Verbesserung kann anhand dieser Methoden im Rahmen einer Cross-Over-Studie bestimmt werden.

Bevorzugt erfolgt die Verwendung des Nahrungsergänzungsmittels durch orale Aufnahme vor dem Schlafengehen. Dabei ist es bevorzugt, dass die Aufnahme kurz vor dem Schlafengehen, beispielsweise 120 Minuten,60 Minuten oder auch 30 Minuten bis unmittelbar vor dem Schlafengehen erfolgt. Das Nahrungsergänzungsmittel kann in diesem Zeitraum kurz vor dem Schlafengehen einmal oder alternativ auch mehrmals aufgenommen werden. Durch die Einnahme des Nahrungsergänzungsmittels kurz vor dem Schlafengehen wird dessen Wirkung auf die im Schlaf ablaufenden Regenerationsprozesse optimiert.

Die Verwendung des Nahrungsergänzungsmittels erfolgt bevorzugt so, dass das Kreatin in einer Tagesdosis von 0,8 g bis 5,0 g, bevorzugter 0,8 g bis 2,0 g aufgenommen wird.

Die Verwendung des Nahrungsergänzungsmittels erfolgt bevorzugt so, dass das Glutamin in einer Tagesdosis von 60 mg bis 5000 mg, bevorzugter 60 mg bis 1000 mg, noch bevorzugter 60 mg bis 200 mg aufgenommen wird.

Die Verwendung des Nahrungsergänzungsmittels erfolgt bevorzugt so, dass das Glycin in einer Tagesdosis von 100 mg bis 500 mg bevorzugter 250 mg bis 400 mg aufgenommen wird.

Sofern das Nahrungsergänzungsmittel Magnesium enthält, erfolgt die Verwendung des Nahrungsergänzungsmittels bevorzugt so, dass das Magnesium in einer Tagesdosis von 40 mg bis 500 mg, bevorzugter 150 mg bis 250 mg aufgenommen wird.

Grundsätzlich ist das Nahrungsergänzungsmittel sowohl für Personen mit Schlafstörungen wie auch für Personen ohne Schlafstörungen ("schlafgesunde" Personen) geeignet. Da das Nahrungsergänzungsmittel, wie oben erläutert, die im Schlaf ablaufenden Regenerationsprozesse beschleunigt und optimiert und somit den Schlaf bzw. den durch die Schlafphase bewirkten Erholungseffekt intensiviert, ist es insbesondere für Personen geeignet, die zwar nicht an krankhaften Schlafstörungen leiden, jedoch einen Schlafmangel (beispielsweise aufgrund beruflicher Belastung etc.) aufweisen, einem Schlafentzug ausgesetzt sind und/oder einen erhöhten Schlafbedarf haben. Mit Schlafmangel ist eine Schlafdauer gemeint, die geringer ist als das durchschnittliche Schlafbedürfnis der betreffenden Personen.

Mit dem Begriff "natürliches Schlafmittel" ist im Rahmen der vorliegenden Erfindung gemeint, dass das Nahrungsergänzungsmittel frei von synthetischen Schlafinittelwirkstoffen ist.

### Beispiele

Bei den folgenden Beispielen 1-4 handelt es sich um erfindungsgemäße Nahrungsergänzungsmittel zur Verwendung bei der Schlafoptimierung, der Verbesserung der Vigilanz und kognitiven Leistungsfähigkeit, der Verbesserung der schlafspezifischen Regeneration, gegen Tagesschläfrigkeit und/oder als natürliches Schlafmittel.

### Beispiel 1

| **Komponente** | | **Menge** | **RDA (%)** |
|---|---|---|---|
| Kreatin | | 1000 mg | |
| Glutamin | | 60 mg | |
| Glycin | | 250 mg | |
| Magnesium | | 150 mg | 75 |
| Vitamin B | Vitamin B1 | 0,6 mg | 50 |
| | Vitamin B2 | 0,7 mg | 50 |
| | Niacin | 8 mg | 50 |
| | Pantothensäure | 3 mg | 50 |
| | Vitamin B6 | 0,7 mg | 50 |
| | Biotin | 25 µg | 50 |
| Zink | | 6 mg | 60 |
| Vitamin C | | 40 mg | 50 |

### Beispiel 2

| **Komponenten** | Menge | R DA (%) |
|---|---|---|
| Vitamin B1 | 0,6 mg | 50 |
| Vitamin B2 | 0,7 mg | 50 |
| Niacin | 8 mg | 50 |
| Vitamin B6 | 0,7 mg | 50 |
| Panthothensäure | 3 mg | 50 |
| Biotin | 25 µg | 50 |
| Vitamin C | 40 mg | 50 |
| Magnesium | 150 mg | 75 |
| Zink | 6 mg | 60 |
| Creatin | 800 mg | - |
| Glutamin | 60 mg | - |
| Glycin | 150 mg | - |
| GABA | 500 mg | - |
| Passionsblume | 250 mg | |
| Hopfenextrakt | 4,8 mg | |

### Beispiel 3

| **Komponente** | | **Menge** | **RDA (%)** |
|---|---|---|---|
| Kreatin | | 1000 mg | |
| Glutamin | | 60 mg | |
| Glycin | | 108 mg | |
| Magnesium | | 45 mg | 15 |
| Vitamin B | Vitamin B1 | 0,9 mg | 75 |
| | Vitamin B2 | 1,1 mg | 75 |
| | Niacin | 12mg | 75 |
| | Pantothensäure | 4,5 mg | 75 |
| | Vitamin B6 | 1,1 mg | 75 |
| | Biotin | 38 µg | 75 |
| Weitere α-Aminosäuren | Alanin | 300 mg | |
| | Arginin | 142 mg | |
| | Asparaginsäure | 720 mg | |
| | Cystein | 142 mg | |
| | Histidin | 125 mg | |
| | Prolin | 410 mg | |
| | Serin | 353 mg | |
| | Threonin | 467 mg | |
| | Tryptophan | 57 mg | |
| | Tyrosin | 131 mg | |
| | Valin | 342 mg | |
| Zink | | 7,5 mg | 75 |
| Vitamin C | | 80 mg | 100 |

### Beispiel 4

| **Komponente** | | **Menge** | **RDA (%)** |
|---|---|---|---|
| Kreatin | | 1500 mg | |
| Glutamin | | 80 mg | |
| Glycin | | 400 mg | |
| Magnesium | | 250 mg | 83 |
| Vitamin B | Vitamin B1 | 1,1 mg | 100 |
| | Vitamin B2 | 1,4 mg | 100 |
| | Niacin | 16 mg | 100 |
| | Pantothensäure | 6 mg | 100 |
| | Vitamin B6 | 1,4 mg | 100 |
| | Biotin | 50 µg | 100 |
| Zink | | 10 mg | 100 |
| Vitamin C | | 80 mg | 100 |

## Patentansprüche

1. Ein Nahrungsergänzungsmittel, enthaltend Kreatin, Glutamin und Glycin, zur Verwendung als natürliches Schlafmittel.

2. Das Nahrungsergänzungsmittel zur Verwendung nach Anspruch 1, weiterhin enthaltend einen oder mehrere Mineralstoffe, bevorzugt Magnesium.

3. Das Nahrungsergänzungsmittel zur Verwendung nach einem der vorstehenden Ansprüche, wobei die Verwendung durch orale Aufnahme vor dem Schlafengehen erfolgt.

4. Das Nahrungsergänzungsmittel zur Verwendung nach einem der vorstehenden Ansprüche, wobei die Verwendung durch Personen erfolgt, die keine krankhafte Schlafstörung aufweisen.

5. Das Nahrungsergänzungsmittel zur Verwendung nach einem der vorstehenden Ansprüche, wobei die Verwendung durch Personen mit Schlafmangel, Schlafentzug und/ oder einem erhöhtem Schlafbedarf erfolgt.

6. Das Nahrungsergänzungsmittel zur Verwendung nach einem der vorstehenden Ansprüche, weiterhin enthaltend ein oder mehrere Vitamine der Vitamin- B-Gruppe.

7. Das Nahrungsergänzungsmittel zur Verwendung nach einem der vorstehenden Ansprüche, weiterhin enthaltend ein Spurenelement, bevorzugt Zink, und/oder gamma-Aminobuttersäure.

8. Das Nahrungsergänzungsmittel zur Verwendung nach einem der vorstehenden Ansprüche bei der Verbesserung der Schafqualität.

9. Das Nahrungsergänzungsmittel zur Verwendung nach einem der vorstehenden Ansprüche bei der Verbesserung der schlafspezifischen Regeneration.

## Claims

1. A food supplement containing creatine, glutamine and glycine to use as a natural sleeping aid.

2. The food supplement to be used according to claim 1, contains one or more mineral substances, particularly magnesium.

3. The food supplement to be used according to one of the preceding claims, is to be consumed by oral intake before going to bed.

4. The food supplement to be used according to one of the preceding claims, is to be used by people who do not suffer from any pathological sleep disorder.

5. The food supplement to be used according to one of the preceding claims, is to be used by people with sleep deprivation and/or increased need for sleep.

6. The food supplement to be used according to one of the preceding claims, contains one or more vitamins of the vitamin B group.

7. The food supplement to be used according to one of the preceding claims, further containing a trace element, particularly zinc, and/or gamma-aminobutyric acid.

8. The food supplement to use according to one of the preceding claims, in improving the quality of sleep.

9. The food supplement to be used according to one of the preceding claims, in improving the sleep specific regeneration.

## Revendications

1. Un complément alimentaire contenant de la créatine, de la glutamine et de la glycine à utiliser comme aide naturelle au sommeil.

2. Le complément alimentaire à utiliser selon la revendication 1, contient un outre une ou plusieurs substances minérales, en particulier du magnésium.

3. Le complément alimentaire à utiliser selon l'une des revendications précédentes, doit être consommé par voie orale avant d'aller au lit.

4. Le complément alimentaire à utiliser selon l'une des revendications précédentes, doit être utilisé par des personnes qui ne souffrent d'aucun trouble pathologique du sommeil.

5. Le complément alimentaire à utiliser selon l'une des revendications précédentes, doit être utilisé par des personnes souffrant d'un manque de sommeil et/ou d'un besoin accru de sommeil.

6. Le complément alimentaire à utiliser selon l'une des revendications précédentes, contient un outre une ou plusieurs vitamines du groupe de la vitamine B.

7. Le complément alimentaire à utiliser selon l'une des revendications précédentes, contient un outre un oligo-élément, en particulier du zinc, et/ou de l'acide gamma-aminobutyrique.

8. Le complément alimentaire à utiliser selon l'une des revendications précédentes, dans l'amélioration de la qualité du sommeil.

9. Le complément alimentaire à utiliser selon l'une des revendications précédentes, dans l'amélioration de la régénération spécifique du sommeil.
